(19) Europäisches Patentamt
European Patent Office
Office européen des brevets

(11) Numéro de publication: **0 118 348**
**A1**

(12) # DEMANDE DE BREVET EUROPEEN

(21) Numéro de dépôt: 84400314.5

(22) Date de dépôt: 15.02.84

(51) Int. Cl.³: C 07 C 103/76
C 07 C 103/78, A 61 K 49/04

(30) Priorité: 23.02.83 FR 8302926

(43) Date de publication de la demande:
12.09.84 Bulletin 84/37

(84) Etats contractants désignés:
AT BE CH DE FR GB IT LI LU NL SE

(71) Demandeur: GUERBET S.A.
16-24 Rue Jean Chaptal
F-93601 Aulnay-sous-Bois Cedex(FR)

(72) Inventeur: Dimo, Ioana
23 Rue du Commandant Mouchotte
F-94160 Saint Mande(FR)

(72) Inventeur: Bonnemain, Bruno
9 Rue de Reims
F-77290 Mitry Mory(FR)

(72) Inventeur: Hardouin, Michel Jean-Charles
88 Avenue Foch
F-94120 Fontenay sous Bois(FR)

(72) Inventeur: Lautrou, Jean
21 Avenue Gambetta
F-94160 Saint Mande(FR)

(74) Mandataire: Bressand, Georges et al,
c/o CABINET LAVOIX 2 Place d'Estienne d'Orves
F-75441 Paris Cedex 09(FR)

(54) **Composés bromés et produits opacifiants en contenant.**

(57) La présente invention a pour objet des composés bromés choisis parmi les composés de formule

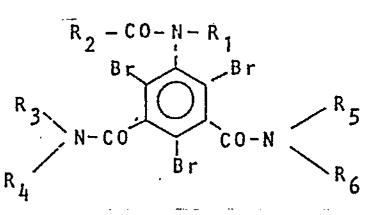

(Ia)

et les composés de formule

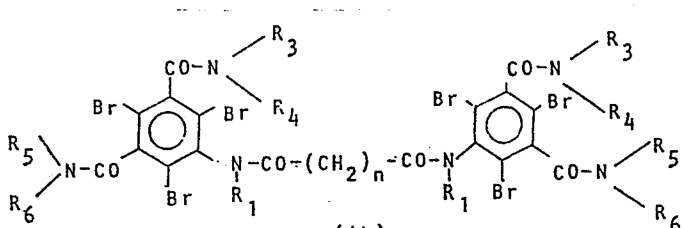

(Ib)

dans lesquelles

$R_1$ représente un atome d'hydrogène ou un groupe alcoyle mono- ou polyhydroxylé,

$R_2$ représente un groupe méthyle, un groupe méthoxy méthyle, un groupe alcoyle mono- ou polyhydroxylé ou un groupe (alcoyl mono- ou polyhydroxylé) carbonylamino méthyle,

$R_3$ et $R_5$ représentent indépendamment l'un de l'autre un groupe alcoyle mono- ou polyhydroxylé, et

$R_4$ et $R_6$ représentent indépendamment l'un de l'autre un atome d'hydrogène, un groupe alcoyle ou un groupe alcoyle mono- ou polyhydroxylé, et

*n* est un nombre entier de 1 à 6.

Ces composés sont utilisables comme produits opacifiants en radiographie, notamment en mélange avec des composés iodobenzéniques.

EP 0 118 348 A1

Composés bromés et produits opacifiants en contenant.-

---

La présente invention concerne des composés utilisables dans des produits opacifiants pour la radiographie.

On utilise depuis longtemps comme produits opacifiants des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont généralement choisis pour conférer aux composés une hydrosolubilité suffisante en vue de l'administration de ces composés en solution aqueuse.

De multiples solutions ont été proposées jusqu'à présent pour augmenter la tolérance des composés iodobenzéniques utilisés comme produits opacifiants.

Un premier type de solution a consisté à synthétiser des structures présentant deux ou trois noyaux benzéniques triiodés (voir par exemple les brevets US-A-3 290 366 et GB 1 346 795).

Un second type de solution a consisté à choisir les substituants autres que les atomes d'iode de façon à obtenir une meilleure tolérance. En particulier, on s'est orienté vers des structures non ioniques, c'est-à-dire ne présentant pas de substituants ionisants tels que des groupes carboxy (voir par exemple les brevets DE-A-2 031 724 et FR-A-2 253 509).

Un troisième type de solution a consisté à synthétiser des composés di- ou tribenzéniques polyiodés dissymétriques présentant un seul groupe ionisant (voir par exemple le brevet US-A-4 014 986).

La Demanderesse a cherché à résoudre le problème de l'augmentation de la tolérance des composés iodobenzéniques d'une manière fondamentalement différente de ce qui a été fait jusqu'à présent.

Elle a découvert que, contrairement à ce que l'on pouvait attendre, si l'on remplace dans les produits opacifiants que l'on utilise en radiographie, en partie les composés iodobenzéniques par des composés bromobenzéniques, non seulement on augmente la tolérance des produits opacifiants, mais on obtient une opacité du même ordre de grandeur.

Ce dernier fait est particulièrement surprenant, car, normalement, on devait s'attendre à ce que le remplacement partiel des composés iodobenzéniques par des composés bromobenzéniques entraîne une diminution notable de l'opacité. En effet, il est connu que l'opacité d'un atome aux rayons X est sensiblement proportionnelle à la puissance 3 de son numéro atomique (J. Duheix, V. Bismuth, M. Laval-Jeantet - Traité de radiodiagnostic, vol. 1 - L'image radiologique, Masson et Cie, 1969). Le numéro atomique du brome est 35, alors que celui de l'iode est 53. L'opacité conférée par le brome devrait donc être trois à quatre fois plus faible. On pouvait donc s'attendre à ce que la présence d'atomes de brome à la place d'une partie des atomes d'iode conduise à une réduction importante de l'opacité. Au contraire, on peut obtenir par le remplacement partiel des composés iodobenzéniques par des composés bromobenzéniques des opacités qui sont du même ordre de grandeur que celles obtenues avec des produits opacifiants ne comprenant que des composés iodobenzéniques. Il s'ajoute à ceci un avantage économique important car le brome est actuellement bien moins onéreux que l'iode.

Jusqu'à présent, on a décrit très peu des composés bromobenzéniques analogues des composés iodobenzéniques utilisés comme produits de contraste.

Elliott C. Lasser dans Amer. J. of Roentg, 1962, 87, 2, 338-360, dans une étude comparative, fait référence à l'analogue bromé de l'acétrizoate de sodium (Urokon), à savoir le tribromo-2,4,6 acétamido-5 benzoate de sodium.

Par ailleurs, le brevet FR-A-1 172 953 décrit des acides aminoisophtaliques halogénés. Bien que le brevet vise essentiellement les dérivés iodés, deux composés bromés sont décrits, à savoir l'acide tribromo-2,4,6 amino-3 isophtalique et l'acide tribromo-2,4,6 acétamido-3 isophtalique.

La présente invention vise à fournir de nouveaux composés bromobenzéniques qui conviennent particulièrement bien en mélange avec des composés iodobenzéniques.

La présente invention a ainsi pour objet des composés choisis parmi les composés de formule

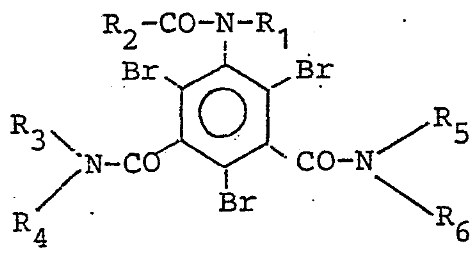

(Ia)

et les composés de formule

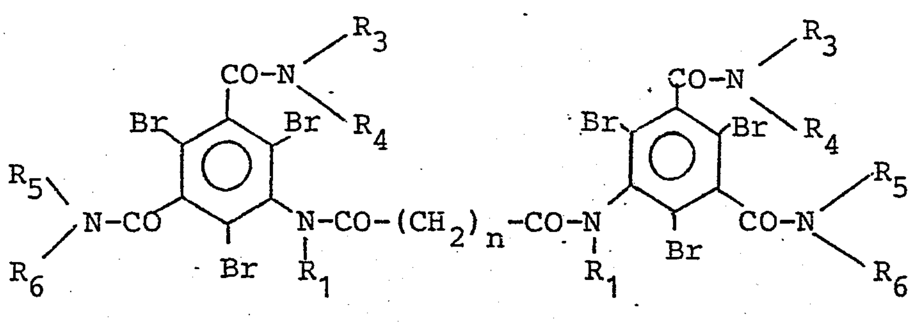

(Ib).

dans lesquelles

$R_1$ représente un atome d'hydrogène ou un groupe alcoyle en $C_1$ à $C_6$ mono- ou polyhydroxylé,

$R_2$ représente un groupe méthyle, un groupe méthoxy méthyle, un groupe alcoyle en $C_1$ à $C_6$ mono- ou polyhydroxylé ou un groupe (alcoyl en $C_{1-6}$ mono- ou polyhydroxylé) carbonylamino méthyle,

$R_3$ et $R_5$ représentent indépendamment l'un de l'autre un groupe alcoyle en $C_1$ à $C_6$ mono- ou polyhydroxylé, et

$R_4$ et $R_6$ représentent indépendamment l'un de l'autre un atome d'hydrogène, un groupe alcoyle en $C_1$ à $C_6$ ou un groupe alcoyle en $C_1$ à $C_6$ mono- ou polyhydroxylé, et

n est un nombre entier de 1 à 6.

Les nouveaux composés bromobenzéniques peuvent être préparés par des procédés classiques et notamment ceux utilisés pour préparer des composés iodobenzéniques.

Les composés peuvent en particulier être préparés par des réactions d'acylation et/ou d'alcoylation de composés à groupe(s) amino.

Les composés de formule I(a) dans lesquels les groupes $-NR_3R_4$ et $-NR_5R_6$ sont identiques peuvent être obtenus à partir du composé de formule

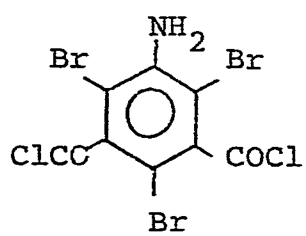

(II)

par acylation avec un chlorure d'acide de formule

$$R'_2-COCl \qquad (III)$$

$R'_2$ étant un groupe $R_2$ dont les groupes hydroxy éventuels sont protégés, conduisant à un dichlorure d'acide de formule

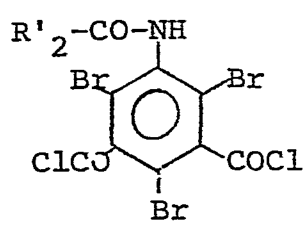

(IVa)

puis condensation de ce chlorure d'acide (IVa) avec une amine de formule

$$R_3R_4NH \qquad (V)$$

conduisant à un composé de formule

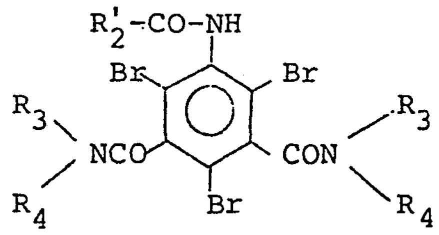

(VIa)

cette réaction étant suivie éventuellement d'une alcoylation avec un dérivé chloré de formule

$$Cl - R_1 \quad (VII)$$

et/ou d'une réaction de déprotection.

En variante, on peut également inverser l'ordre des réactions, c'est-à-dire notamment effectuer d'abord une condensation du chlorure d'acide (II) avec une amine de formule (V) conduisant à une amine de formule

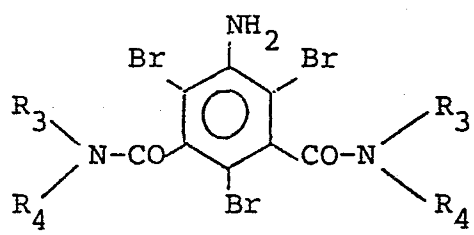

(VIII a)

puis une acylation de l'amine avec un chlorure d'acide de formule (III), cette réaction étant suivie éventuellement d'une alcoylation avec un dérivé de formule (VII) et/ou d'une réaction de déprotection.

Les composés de formule (Ia) dans lesquels les groupes $-NR_3R_4$ et $-NR_5R_6$ sont différents peuvent être obtenus à partir d'un composé de formule

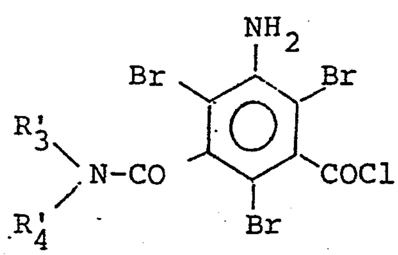

(IX)

$R'_3$ et $R'_4$ étant des groupes $R_3$ et $R_4$ dont les groupes hydroxy sont protégés par acylation avec un chlorure d'acide de formule (III) conduisant à un chlorure d'acide de formule

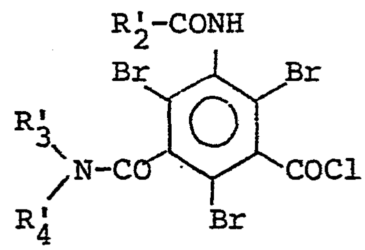

(Xa)

puis condensation de ce chlorure d'acide (Xa) avec une amine de formule $R_5R_6NH$ (XI), conduisant à un composé de formule

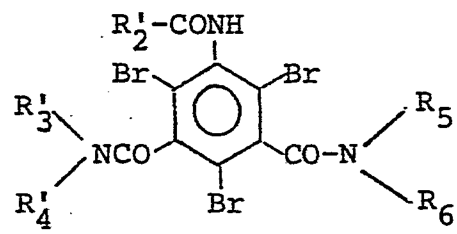

(XIIa)

cette réaction étant suivie éventuellement d'une alcoylation avec un dérivé chloré de formule (VII) et/ou d'une réaction de déprotection.

En variante, on peut également inverser l'ordre des réactions.

En particulier, on peut effectuer d'abord une condensation d'un composé de formule IX avec une amine de formule XI conduisant à une amine de formule

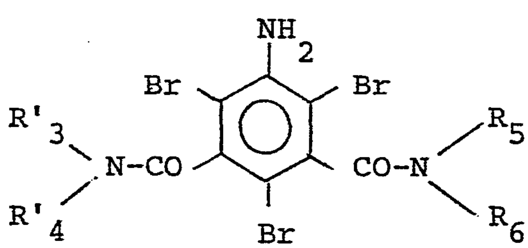

(XIII)

puis une acylation de l'amine ainsi obtenueavec un chlorure d'acide de formule III conduisant à un composé de formule XIIa.

On effectue ensuite éventuellement une alcoylation du composé de formule XIIa avec un dérivé chloré de formule VII et/ou une réaction de déprotection.

On peut également effectuer d'abord une acylation d'un composé de formule II avec un chlorure d'acide de formule III conduisant à un dichlorure d'acide de formule IVa, puis successivement une condensation avec une amine de formule V et une condensation avec une amine de formule XI

Les composés de formule (Ib) peuvent être préparés de façon similaire aux composés de formule (Ia).

Ainsi, les composés de formule (Ib) dans laquelle les groupes $-NR_3R_4$ et $-NR_5R_6$ sont identiques peuvent être obtenus à partir du composé de formule (II) par acylation avec un chlorure d'acide de formule

$$Cl-CO-(CH_2)_n-CO-Cl \qquad \text{(IIIb)}$$

conduisant à un chlorure d'acide de formule

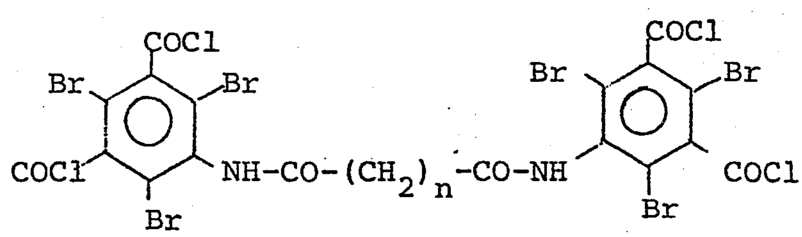

(IVb)

puis condensation de ce chlorure d'acide (IVb) avec une amine de formule (V) conduisant à un composé de formule

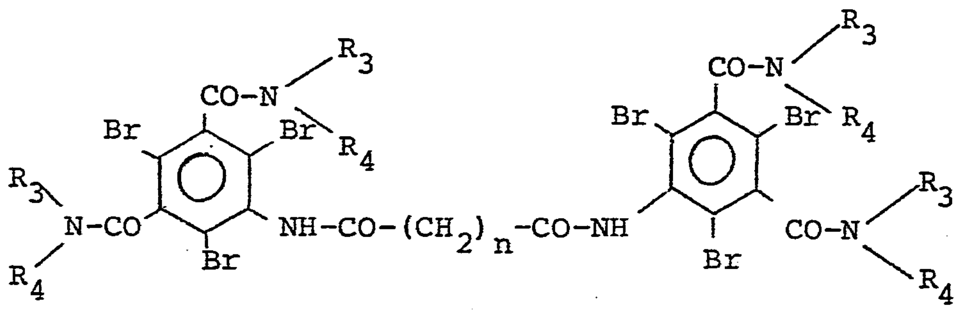

(VIb)

cette réaction étant suivie éventuellement d'une alcoylation avec un dérivé chloré de formule (VII).

De même, les composés de formule (Ib) dans lesquels les groupes $-NR_3R_4$ et $-NR_5R_6$ sont différents peuvent être obtenus à partir d'un composé de formule (IX) par condensation avec un chlorure d'acide de formule (IIIb), puis condensation du chlorure d'acide obtenu avec une amine de formule (XI), cette réaction étant suivie éventuellement d'une alcoylation avec un dérivé chloré de formule (VII) et/ou d'une réaction de déprotection.

La présente invention a également pour objet des produits opacifiants pour la radiographie, présentant une tolérance plus grande que celle des produits opacifiants comprenant uniquement des composés iodobenzéniques et une opacité du même ordre de grandeur que ces produits opacifiants, caractérisé en ce qu'ils comprennent des mélanges de composés iodobenzéniques et de composés bromobenzéniques de formule (Ia) ou (Ib).

Dans ces produits opacifiants, les composés bromobenzéniques représentant avantageusement de 1/2 à 2/1

(en moles) des composés iodobenzéniques. Dans les mélanges, les composés iodobenzéniques peuvent être notamment des composés de structure analogue à celle des composés de formule (Ia) ou (Ib), c'est-à-dire des composés de formule

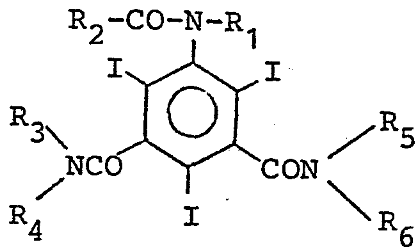

(XIIIa)

ou de formule

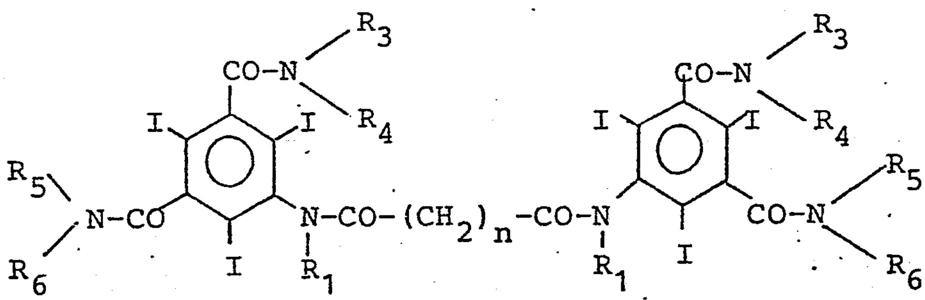

dans lesquelles $R_1$, $R_2$, $R_3$, $R_4$, $R_5$, $R_6$ et n ont les significations données pour la formule (I).

Comme exemples de tels composés, on peut citer le 5-(N-2,3-dihydroxypropyl-N-acétylamino)-2,4,6-triiodo-N,N'-bis (2,3-dihydroxypropyl) isophtalamide et le 5-(N-2-hydroxypropionyl-amino)-2,4,6-triiodo-N,N'bis (1,3-dihydroxy-2-propyl) isophtalamide.

Les mélanges peuvent également comprendre comme composés iodobenzéniques des composés appartenant aux classes suivantes :

1 - Les composés de formule

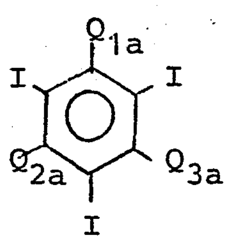

(XIV)

dans laquelle

$Q_{1a}$ est un groupe -COOH (généralement salifié par une base pharmacologiquement acceptable),

$Q_{2a}$ représente un atome d'hydrogène, un radical de formule 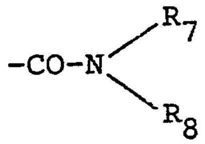, $R_7$ et $R_8$ étant indépendamment l'un de l'autre un atome d'hydrogène, un radical alcoyle inférieur, hydroxyalcoyle inférieur ou alcanoyl (inf.) oxyalcoyle (inf.), un groupe amino ou un radical de formule 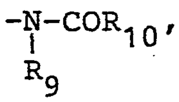, $R_9$ étant un radical alcoyle inférieur, hydroxy-alcoyle inférieur ou alcoxy (inf.) alcoyle (inf.) et $R_{10}$ un atome d'hydrogène, un radical alcoyle inférieur ou hydroxyalcoyle inférieur,

$Q_{3a}$ représente un groupe amino ou un radical de formule 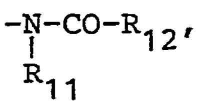, dans laquelle $R_{11}$ et $R_{12}$ ont les significations données respectivement pour $R_9$ et $R_{10}$.

2 - Les composés de formule

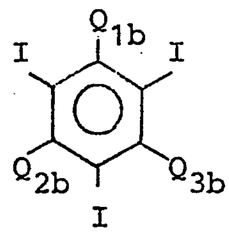

(XV)

dans laquelle

$Q_{1b}$ représente un radical de formule $-\underset{R_{14}}{\underset{|}{N}}-COR_{13}$,

$R_{13}$ étant un radical alcoyle(inf) polyhydroxylé

$R_{14}$ étant un atome d'hydrogène ou un radical alcoyle inférieur

$Q_{2b}$ représente un radical de formule $-CO-N{\nearrow R_{15} \atop \searrow R_{16}}$

dans laquelle $R_{15}$ et $R_{16}$ ont les singifications données respe c tivement pour $R_7$ et $R_8$

$Q_{3b}$ représente un radical de formule $-CO-N{\nearrow R_{17} \atop \searrow R_{18}}$

dans laquelle $R_{17}$ et $R_{18}$ ont les significations données respectivement pour $R_7$ et $R_8$ ou un radical de formule $-\underset{R_{20}}{\underset{|}{N}}-CO-R_{19}$ dans laquelle $R_{19}$ et $R_{20}$ ont les significations données respectivement pour $R_9$ et $R_{10}$.

3 - Les composés de formule

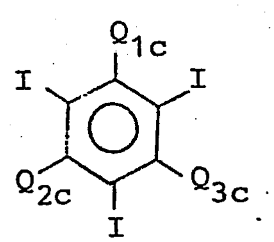

(XVI)

dans laquelle

$Q_{1c}$ représente un radical de formule $-CONH-R_{21}$ dans

laquelle $R_{21}$ est un reste de sucre ou un radical alcoyle(inf) polyhydroxylé

$Q_{2c}$ représente un radical $-\underset{R_{23}}{N}-COR_{22}$, dans laquelle $R_{22}$ et $R_{23}$ ont les significations données respectivement pour $R_9$ et $R_{10}$ , et

$Q_{3c}$ représente un radical de formule $-CO-N\begin{matrix}R_{24}\\R_{25}\end{matrix}$ , dans laquelle $R_{24}$ et $R_{25}$ ont les significations données respectivement pour $R_7$ et $R_8$, ou un radical de formule $-\underset{R_{27}}{N}-CO-R_{26}$ , dans laquelle $R_{26}$ et $R_{27}$ ont les significations données respectivement pour $R_9$ et $R_{10}$.

4 - Les composés de formule

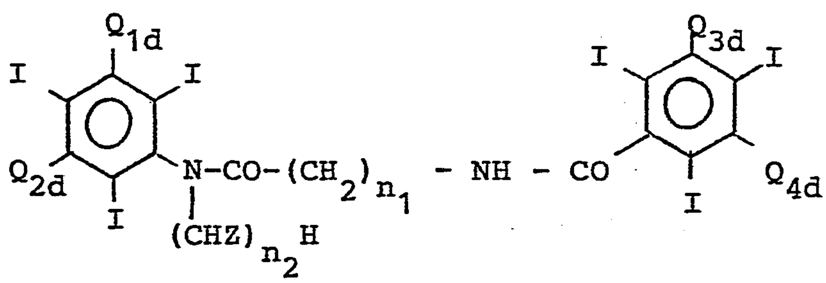

(XVII)

dans laquelle

$Q_{1d}$ est un groupe -COOH (généralement salifié par une base pharmacologiquement acceptable).

$Q_{2d}$ et $Q_{3d}$ sont des radicaux ayant les mêmes significations que $Q_{2a}$, $Q_{2d}$ pouvant également représenter un radical $-CH_2OH$

$Q_{4d}$ représente un groupe $-NH_2$ ou un radical de formule $-\underset{R_{29}}{N}-CO-R_{28}$ dans laquelle $R_{28}$ a la signification donnée pour $R_9$ et $R_{29}$ a la signification donnée pour $R_{10}$ ou est un radical alcanoyle inférieur,

Z est H ou OH

$n_1$ = 1 à 3
$n_2$ = 0 à 6.

5 - les composés de formule

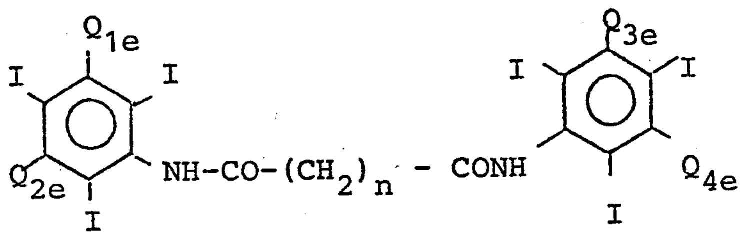

(XVIII)

dans laquelle

$Q_{1e}$ et $Q_{3e}$ sont des groupes COOH (généralement salifiés par des bases pharmacologiquement acceptables)

$Q_{2e}$ et $Q_{4e}$ ont les significations données pour $Q_{2a}$.

6 - Les composés de formule

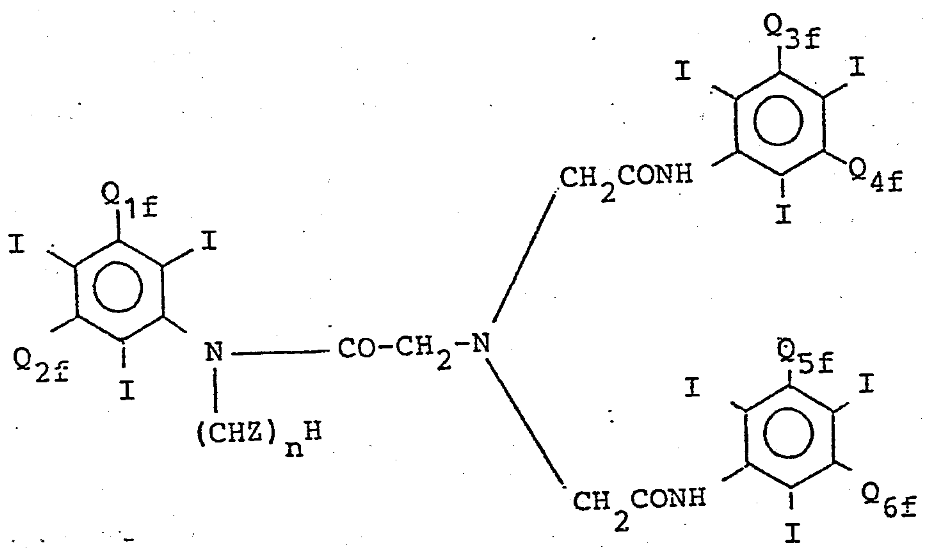

(XIX)

dans laquelle

$Q_{1f}$ est un groupe COOH (généralement salifié par une base pharmacologiquement acceptable)

$Q_{2f}$, $Q_{3f}$, $Q_{4f}$, $Q_{5f}$ et $Q_{6f}$ ont les significations données pour $Q_{2a}$, Z est H ou OH et n = 0 à 6.

7 - les composés de formule

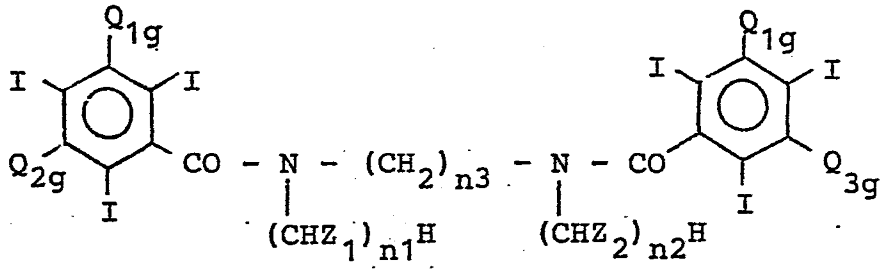 (XX)

dans laquelle

$Q_{1g}$ représente un radical de formule - N - $COR_{30}$
$R_{31}$

$R_{30}$ étant un radical alcoyle(inf) polyhydroxylé

$R_{31}$ étant un atome d'hydrogène ou un radical alcoyle inférieur,

$Q_{2g}$ et $Q_{3g}$ représentent indépendamment l'un de l'autre un radical de formule

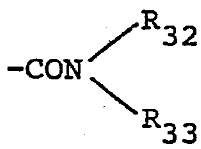 , $R_{32}$ et $R_{33}$ étant indépendamment l'un de l'autre un atome d'hydrodrogène, un radical alcoyle inférieur, hydroxyalcoyl inférieur ou alcanoyl (inf) oxyalcoyle (inf), un groupe amino ou un radical de formule
-N-$COR_{34}$
$R_{35}$

$R_{34}$ étant un radical alcoyle inf., hydroxy alcoyle inférieur ou alcoxy (inf) alcoyle (inf) et $R_{35}$ un atome d'hydrogène, un radical alcoyle inférieur ou hydroxyalcoyle inférieur,

$Z_1$ et $Z_2$ sont H ou OH

$n_1$ et $n_2$ = 0 à 6

$n_3$ = 0 à 4.

8 - Les composés de formule

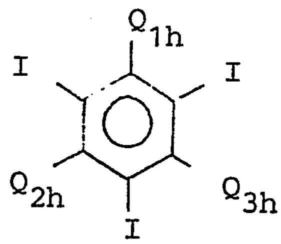

(XXI)

dans laquelle

$Q_{1h}$, $Q_{2h}$ et $Q_{3h}$ représentent indépendamment l'un de l'autre un groupe -COOH, un groupe -COOH salifié par une base pharmacologiquement acceptable ou un groupe de formule 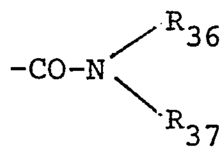, dans laquelle $R_{36}$ et $R_{37}$ représentent indépendamment l'un de l'autre un atome d'hydrogène, un radical alcoyle inférieur, hydroxyalcoyle(inf.) ou alcanoyl (inf.) oxyalcoyle (inf.).

Dans les définitions des formules (XIV) à (XXI) le terme "inférieur" appliqué aux radicaux alcoyle, alcoxy ou alcanoyle désigne généralement des radicaux ayant de 1 à 6 atomes de carbone; de plus, par radical hydroxyalcoyle, on désigne un radical alcoyle mono- ou polyhydroxylé.

Les composés iodobenzéniques ont été largement décrits dans la littérature et un certain nombre d'entre eux sont commercialisés.

Comme exemples de composés iodobenzéniques de formule (XIV), on peut citer l'acide diatrizoïque, l'acide iothalamique, l'acide métrizoïque, l'acide acétrizoïque, l'iodamide et l'acide ioxitalamique.

Comme exemple de composés iodobenzéniques de formule (xv), on peut citer le ioglunide.

Comme exemple de composés iodobenzéniques de formule (XVI), on peut citer le métrizamide.

Comme exemple de composés iodobenzéniques de formule (XVII), on peut citer l'acide ioxaglique.

Comme exemples de composés iodobenzéniques de formule (XVIII), on peut citer l'acide iocarmique, l'adipiodone et l'acide ioglycamique.

Les composés bromobenzéniques de formule (I) peuvent en outre être utilisés en radiographie numérisée telle que l'angiographie numérisée.

La présente invention a donc également pour objet des produits opacifiants qui comprennent des composés de formule (I).

La forme pharmaceutique préférée des produits opacifiants selon l'invention est constituée par des solutions aqueuses de composés de formule (I) en mélange ou non avec des composés iodobenzéniques.

Les solutions aqueuses contiennent généralement un total de 5 à 100 g de composés bromobenzéniques et éventuellement de composés iodobenzéniques pour 100 ml et la quantité injectable de telles solutions peut varier généralement de 5 à 1000 ml.

On donnera ci-après des exemples de compositions selon la présente invention.

<u>Composition A</u>

| | |
|---|---|
| 5-(N-2,3-dihydroxypropyl-N-acétyl-amino)-2,4,6-triiodo-N,N'-bis (2,3-dihydroxy-propyl) isophtalamide | 30 g |
| Composé de l'exemple 1 | 25 g |
| Eau pour préparation injectable Q.S.P. | 100 ml |

<u>Composition B</u>

| | |
|---|---|
| 5-(N-2,3-dihydroxypropyl-N-acétyl-amino)-2,4,6-triiodo-N,N'-bis (2,3-dihydroxy-propyl) isophtalamide | 30 g |
| Composé de l'exemple 2 | 26 g |
| Eau pour préparation injectable Q.S.P. | 100 ml |

<u>Composition C</u>

| | |
|---|---|
| Ioxaglate de méthylglucamine | 30 g |
| Composé de l'exemple 1 | 25 g |
| Eau pour prépration injectable Q.S.P. | 100 ml |

Les exemples suivants illustrent la préparation des composés de formule (I).

EXEMPLE 1

Préparation du 5-(N-2,3-dihydroxypropyl N-acétylamino)-2,4,6-tribromo-N,N'-bis (2,3-dihydroxypropyl) isophtalamide

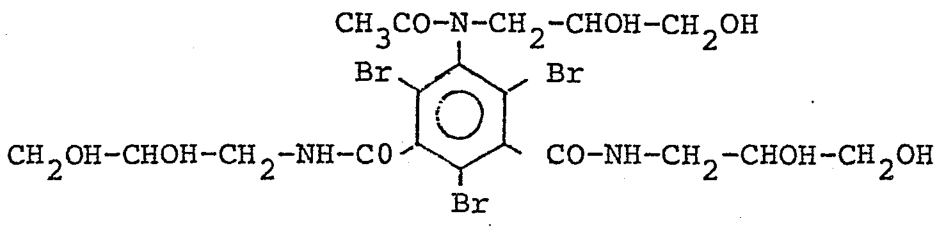

a) Préparation du chlorure de l'acide 5-amino-2,4,6-tribromo isophtalique

500 g de l'acide 5-amino-2,4,6-tribromo isophtalique (1,2 mole) sont mis en suspension avec 3 l de chlorure de thionyle et 1 ml de diméthyl formamide. Le mélange est porté au reflux pendant 4 heures.

Après évaporation du chlorure de thionyle en excès, le produit est lavé dans 2 litres d'éther diisopropylique. La totalité du produit est précipitée par addition d'éther de pétrole. Le dichlorure est filtré, puis claircé à l'éther de pétrole et séché sous vide. (Rdt. 90 %).

CCM : Butanol/eau/acide acétique (50/25/11) Rf: 0,9.

b) Préparation du chlorure de l'acide 5-acétamido tribromo-2,4,6 isophtalique

25,6 ml (0,36 mole) de chlorure d'acétyle sont ajoutés goutte à goutte à une solution contenant 110 g (0,24 mole) de chlorure d'acide 5-amino 2,4,6-tribromo isophtalique dans 120 ml de diméthylacétamide à 0°C.

La solution est chauffée 2 heures à 40°C, puis agitée à température ambiante. Le produit blanc qui cristallise peu à peu est essoré après 16 h, lavé à l'eau additionnée de quelques gouttes de soude jusqu'à pH 7, puis lavé à l'éther isopropylique. Rdt. 39,5 %. Un deuxième jet est obtenu par précipitation des eaux mères à l'eau glacée ce qui donne un rendement global de 83%.

CCM : le produit condensé à la propylamine migre à un

Rf de 0,6 dans l'éluant Benzène/méthyléthylcétone/acide formique (50/25/20)

Structure confirmée par spectre IR et RMN.

c) Préparation du 5-acétamido-2,4,6-tribromo-N,N'-bis-(2,3-dihydroxypropyl) isophtalamide

40 g (0,080 mole) du chlorure de l'acide 5-acétamido-2,4,6-tribromo isophtalique dissous dans 40 ml d'acétone sont ajoutés goutte à goutte dans une solution de 3-aminopropane-1,2-diol (0,24 mole) dans 40 ml d'eau en présence de 0,24 mole de bicarbonate de potassium. On agite 16 heures à température ambiante. On essore le produit qui a cristallisé et on le lave trois fois à l'éthanol afin d'éliminer l'excès d'aminopropanediol.

Puis le produit est dissous au reflux dans le méthanol, les sels minéraux sont éliminés par filtration à chaud et le produit purifié recristallise dans le méthanol.

CCM : Butanol/eau/$CH_3COOH$ (50/25/11). Rf : 0,35.
Isobutanol/isopropanol/ammoniaque 28 % (35/35/40) Rf : 0,3
Méthanol/chloroforme (40/60) Rf : 0,57.

La structure est confirmée par les spectres IR et $RMN^1H$.

d) Préparation du 5-(N-2,3-dihydroxypropyl-N-acétylamino)-2,4,6-tribromo-N,N'-bis-(2,3-dihydroxypropyl) isophtalamide

13 millimoles de méthylate sont préparés à partir de 13 millimoles de sodium dans 12 ml de méthanol anhydre. On ajoute 13 ml d'éthylène glycol sec et on y dissout 7,9 g (13 millimoles) de 5-acétamido-2,4,6-tribromo-N,N'-bis (2,3-dihydroxypropyl) isophtalamide.

On chauffe 15 mn à 50°C puis on évapore l'excès de méthanol sous vide. On ajoute alors goutte à goutte 13,5 millimoles de 3-chloropropane-1,2-diol. On chauffe 5 heures à 50°C. On filtre un insoluble correspondant à 50 % du chlorure de sodium formé dans la réaction, puis on achève la déminéralisation sur résine cationique

$H^+$ forte (IRN 77) puis sur résine anionique $OH^-$ (IRN 78).

CCM : Butanol/eau/$CH_3COOH$ (50/25/11) Rf : 0,30
Isobutanol/isopropanol/ammoniaque (35/35/40) Rf:0,30
Méthanol/chloroforme (40/60) Rf : 0,43.

EXEMPLE 2

Préparation du 5-(N-2,3-dihydroxy propyl-N-méthoxyacétyl-amino)-2,4,6-tribromo-N,N'-bis (2,3-dihydroxypropyl) isophtalamide

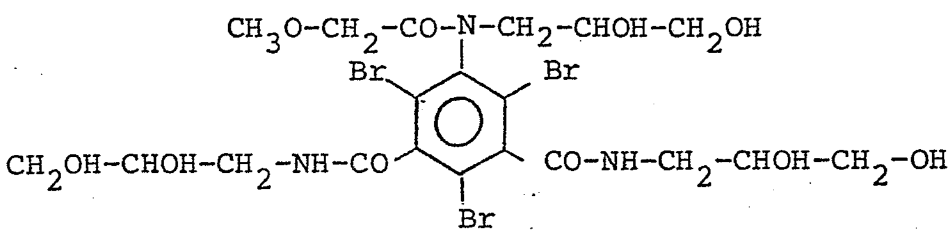

a) Préparation du chlorure de l'acide-5-(N-méthoxyacétyl-amino)-2,4,6-tribromo isophtalique

On ajoute goutte à goutte et sous agitation à une température inférieure à 10°C, 22,7 g (0,05 mole) de chlorure d'acide 5-amino-2,4,6-tribromo isophtalique en suspension dans 75 ml de $CHCl_3$ en présence de 8 ml (0,055 mole) de triéthylamine, sur une solution chloroformique contenant 0,25 mole de chlorure d'acide méthoxy acétique: celui-ci est fabriqué in situ dans $CHCl_3$ à partir de 18,7 ml (0,25 mole) d'acide méthoxyacétique et 18 ml (0,25 mole) de chlorure de thionyle par agitation 1 heure à température inférieure à 10°C.

Le mélange réactionnel est chauffé à reflux du chloroforme une journée jusqu'à disparition du produit de départ. Après lavage à l'eau bicarbonatée, séchage et évaporation de la phase chloroformique, on obtient 19,6 g d'un solide blanc, soit un rendement de 74,5 %, avec un point de fusion net F : 145°C.

CCM: sur le produit condensé avec la propylamine
Isopropanol/acétate d'éthyle/$NH_4OH$ (35/35/40) RF: 0,98.

b) Préparation du 5-(N-méthoxyacétyl-amino)-2,4,6-tribromo-N,N'-bis (2,3-dihydroxypropyl)-isophtalamide

42 g (0,08 mole) du chlorure de l'acide 5-méthoxy-acétyl-amino-2,4,6-tribromo isophtalique en solution dans 40 ml d'acétone sont ajoutés goutte à goutte à une solution de 0,24 mole de 3-aminopropane-1,2-diol dans 40 ml d'eau en présence de 0,24 mole de bicarbonate de potassium. Le mélange réactionnel est agité 16 heures à température ambiante. Le produit cristallisé est essoré, puis lavé trois fois à l'éthanol.

Après dissolution du produit au reflux du méthanol, suivie d'une filtration à chaud, afin d'éliminer les sels minéraux, le produit est purifié par recristallisation dans le méthanol.

CCM : Butanol/eau/$CH_3COOH$ (50/25/11) Rf : 0,46

Benzène/MEC/HCOOH (60/25/10) Rf : 0,04

La structure est confirmé par spectrométrie IR et $^1$HRMN.

c) <u>Préparation du 5-(N-2,3-dihydroxypropyl-N-méthoxy acétyl-amino)-2,4,6-tribromo-N,N'-bis (2,3-dihydroxy-propyl) isophtalamide</u>

8,3 g (13 millimoles) du 5-(N-méthoxyacétyl-amino)-2,4,6-tribromo-N,N'-bis (2,3-dihydroxypropyl) isophtalamide sont dissous dans un mélange d'éthylène glycol anhydre (13 ml) et de méthylate de sodium (13 millimoles).

Le mélange réactionnel est chauffé 15 minutes à 50°C, l'excès de méthanol est évaporé sous vide.

13,5 millimoles de 3-chloro propane-1,2-diol sont ensuite ajoutés goutte à goutte. Dès la fin de l'addition le mélange est chauffé à 50°C pendant 5 heures. Les sels minéraux (NaCl) sont éliminés par filtration et par passages successifs sur une résine cationique (IRN 77) et une résine anionique (IRN 78).

CCM : Isobutanol/isopropanol/ammoniaque (35/35/40) Rf : 0,48

Butanol/eau/$CH_3COOH$ (50/25/11) Rf : 0,53

Méthanol/chloroforme (40/60) Rf : 0,66

<u>Exemple 3</u>

Préparation du 5-(N-méthylacétyl-amino)-2,4,6-tribromo-N,N'-bis (2-méthyl-1,3-dihydroxy-2-propyl) isophtalamide

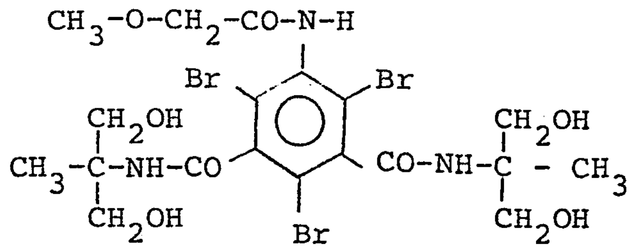

Un mélange contenant 3 ml (0,022 mole) de triéthylamine et 2,1 g (0,02 mole) de 2-amino 2-méthyl propane-1,3-diol dissous dans 15 ml de DMAC est additionné goutte à goutte à température ambiante sur une solution contenant 5,3 g (0,01 mole) du dichlorure de l'acide 2,4,6-tribromo-5-(N-méthoxyacétyl-amino) isophtalique obtenu à l'exemple 2(a) dissous dans 15 ml de DMAC. L'agitation est maintenue une nuit à 40°C.

Après filtration du précipité et lavages successifs au chloroforme et à l'éther de pétrole, on obtient 5,5 g d'un produit solide (rendement: 83 %).

CCM : acétone/$CHCl_3$/acide acétique (50/40/10) Rf : 0,25.

EXEMPLE 4

Préparation du L-5-(N-2-hydroxypropionyl-amino)-2,4,6-tribromo-N,N'-bis (2-méthyl-1,3-dihydroxy-2-propyl) isophtalamide

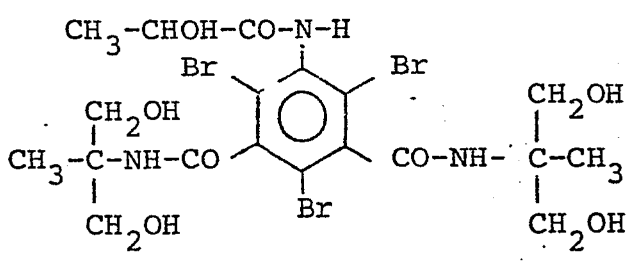

a) Préparation de l'acide L-5α-acétoxypropionyl-amino-2,4,6-tribromo isophtalique

On ajoute sous agitation à température ambiante 24 g (0,16 mole) de chlorure de L-2-acétoxypropionyle dissous dans 10 ml de DMAC à 22,8 g (0,05 mole) de dichlorure d'acide 5-amino-2,4,6-tribromo isophtalique dissous dans 50 ml de DMAC.

Le mélange est agité à 20°C jusqu'à disparition du produit de départ puis versé sur 1,5 l d'eau.

Après 18 heures d'agitation le précipité est filtré, puis lavé à l'éther de pétrole. On purifie ce produit par solubilisation dans l'éther et lavage à l'eau.

Après séchage et évaporation, on obtient 20 g de solide crème, soit un rendement de 70 %.

CCM : Butanol/$H_2O$/acide acétique (50/25/11) Rf : 0,90.

b) Préparation du L-5-(N-2-acétoxypropionyl-amino)-2,4,6-tribromo-N,N'-bis (2-méthyl-1,3-dihydroxy-2-propyl) isophtalamide

23 g (0,04 mole) du composé obtenu en (a) dissous dans 100 ml de DMAC sont ajoutés lentement à un mélange de 8,4 g (0,08 mole) de 2-amino 2-méthyl propane-1,3-diol et 13,7 ml (0,10 mole) de triéthylamine dans 25 ml de DMAC.

On observe un léger effet thermique lors de l'addition (la température passe de 20 à 30°C). Après quelques heures, la réaction est terminée.

Le précipité de chlorhydrate de triéthylamine est filtré. Le mélange est évaporé , puis versé sur 250 ml de $CHCl_3$. Le précipité beige est filtré, puis relavé dans l'éther. Après filtration et séchage on obtient un produit solide. Rendement : 80 %.

CCM : Butanol/eau/$CH_3COOH$ (50/25/11) Rf : 0,7
Toluène/MEC/HCOOH (60/25/25) Rf : 0,20

c) Préparation du L-5-(N-2-hydroxypropionyl-amino)-2,4,6-tribromo-N,N'-bis (2-méthyl-1,3-dihydroxy-2-propyl) isophtalamide

On dissout le composé obtenu en (b) dans l'eau et on amène son pH à 11 par addition de soude caustique concentrée ( 0,013 mole ) puis on chauffe à 40°C et on ajoute de l'hydroxyde de sodium jusau'à pH constant.

La solution est déminéralisée par passage sur résine anionique, puis évaporée jusqu'à siccité.

On obtient un produit solide. Rendement : 80 %.

CCM : Butanol/eau/$CH_3COOH$ (50/25/11) Rf : 0,8.

EXEMPLE 5

Préparation du L-5-(N-2-hydroxypropionyl-amino)-2,4,6-tribromo-N,N'-bis (2,3-dihydroxypropyl) isophtalamide

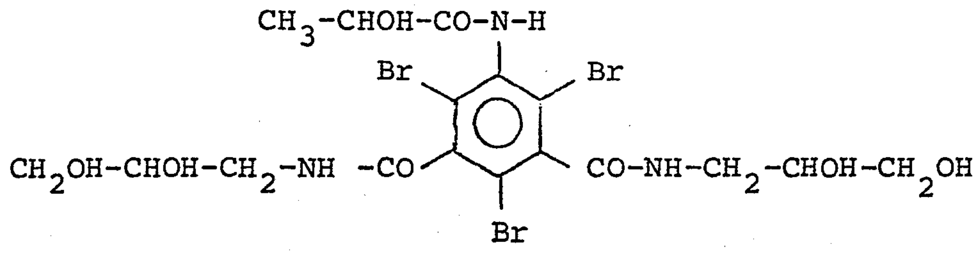

a) Préparation du L-5-(N-2-acétoxypropionyl-amino)-2,4,6-tribromo-N,N'-bis (2,3-dihydroxypropyl) isophtalamide

On opère comme à l'exemple 4(b) en utilisant à la place du 2-amino-2-méthylpropane-1,3-diol le 3-aminopropane-1,2-diol.

On obtient un produit solide. Rendement : 86 %.

CCM : Butanol/eau/$CH_3COOH$ (50/25/11) Rf : 0,55.

b) Préparation du L-5-(N-2-hydroxypropionyl-amino)-2,4,6-tribromo-N,N'-bis (2,3-dihydroxypropyl) isophtalamide

On traite le composé obtenu en (a) comme décrit à l'exemple 4(b).

On obtient un produit solide. Rendement : 75 %.

CCM : Butanol/eau/$CH_3COOH$ (50/25/11) Rf : 0,40.

EXEMPLE 6

Préparation du L-5-(N-2-hydropropionyl-amino)-2,4,6-tribromo-N,N'-bis /bis (β-hydroxyéthyl)7 isophtalamide

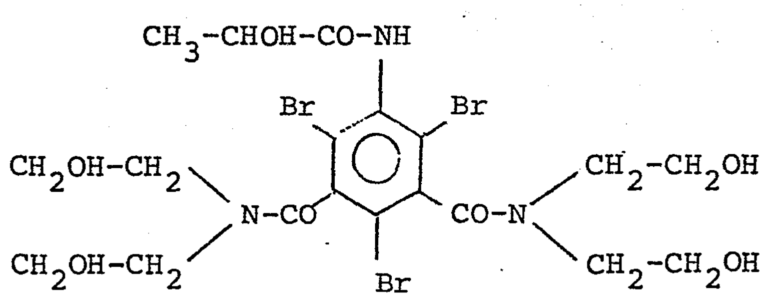

a) Préparation du 5-amino-2,4,6-tribromo-N,N'-bis /bis(β-hydroxyéthyl)/ isophtalamide

368 g du chlorure de l'acide 5-amino-2,4,6-tribromo isophtalique (Exemple 1(a) sont dissous dans 380 ml d'acétone. De la diéthanolamine (236 ml) en solution dans 200 ml d'eau et 236 g d'hydrogénocarbonate de potassium sont ajoutés lentement en maintenant la température du mélange réactionnel aux environs de 20°C.

Après plusieurs jours d'agitation à température ambiante, le produit cristallise.

Après filtration et séchage sous vide, le produit est recristallisé dans 1500 ml d'éthanol absolu.
Rendement 70 %.
CCM : Butanol/eau/acide acétique (50/25/11) Rf: 0,55 et 0,65.

b) Préparation du L-5-(N-2-acétoxypropionyl-amino)-2,4,6-tribromo-N,N'-bis /bis (β-hydroxyéthyl)/ isophtalamide

A une solution de 32,5 g (0,0553 mole) de 5-amino-2,4,6-tribromo-N,N'-bis /bis (β-hydroxyéthyl)/ isophtalamide dans 55 ml de DMAC, on ajoute goutte à goutte 25 g (0,166 mole) de chlorure d'acide L-α-acétoxypropionique dissous dans 10 ml de DMAC.

Après agitation pendant 48 heures, à température ambiante, on verse le mélange sur 1,5 l d'eau. L'huile obtenue est extraite par $CHCl_3$. La phase organique est lavée, séchée,puis évaporée. On obtient une huile.
CCM : Isobutanol/isopropanol/ammoniaque (35/35/40) Rf: 0,75.

c) Préparation du L-5-(N-2-hydroxypropionyl-amino)-2,4,6-tribromo-N,N'-bis (2,3-dihydroxypropyl) isophtalamide

Le composé obtenu en (b) (0,0553 mole) est saponifié par 1,3 équivalent de soude aqueuse 2N. Après agitation pendant 48 heures à température ambiante, on passe successivement sur résine cationique (IRN 77), puis anionique (IRN 78).

CCM : Isopropanol/isobutanol/ammoniaque (35/35/40) Rf: 0,55.

EXEMPLE 7

Préparation du 5-/N̄-2,2-bis-(hydroxyméthyl) propionyl-amino/ 2,4,6-tribromo-N,N'-bis /bis (β-hydroxyéthyl)/ isophtalamide

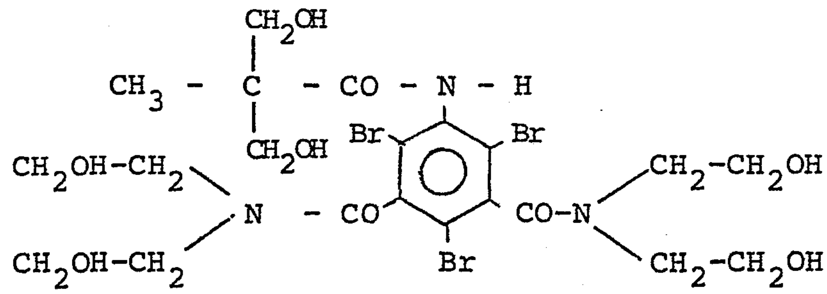

a) Préparation du 5-(N-2,2-bis-acétoxyméthyl propionyl-amino)-2,4,6-tribromo-N,N'-bis-/bis (β-hydroxyéthyl)/ isophtalamide

Il est obtenu comme décrit à l'exemple 6(b) en utilisant comme chlorure d'acide, le chlorure de l'acide 2,2-bis acétoxypropionyle.

CCM : Isobutanol/isopropanol/ammoniaque (35/35/40) Rf : 0,65.

b) Préparation du 5-/N̄-2,2-bis(hydroxyméthyl) propionyl-amino/-2,4,6-tribromo-N,N'-bis /bis (βhydroxyéthyl)/ isophtalamide

Il est obtenu par saponification du composé obtenu en (a) comme décrit à l'exemple 6(c).

CCM : Isobutanol/isopropanol/ammoniaque (35/35/40) Rf: 0,5

EXEMPLE 8

Préparation du 5-(N-méthoxyacétyl-amino)-2,4,6-tribromo-N-bis-(β-hydroxyéthyl)-N'-(2-méthyl-1,3-dihydroxy-2-propyl) isophtalamide

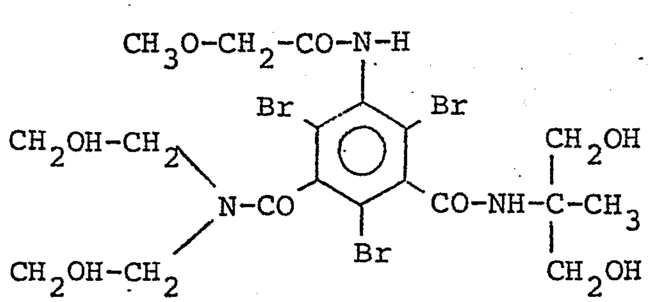

a) Préparation du chlorure d'acide 3-N-bis-(acétoxyéthyl) carbamoyl-5-méthoxy acétyl-amino-2,4,6-tribromo-isophtalique

Sur 0,027 mole de chlorure d'acide méthoxyacétique fabriqué in situ dans le DMAC anhydre, on ajoute goutte à goutte, à une température inférieure à 5°C, 5,4 g (0,009 mole) de chlorure d'acide 3-N-diacétoxyéthylcarbamoyl-5-amino-2,4,6-tribromo-isophtalique.

Après agitation 48 h à température ambiante, la solution est versée sur 100 ml d'eau. L'huile obtenue est extraite au $CHCl_3$, lavée à l'eau bicarbonatée, puis séchée et évaporée. On obtient une huile.

CCM : Toluène/méthyléthyl cétone/acide formique (60/25/25)

Rf : 0,75.

b) Préparation de 5-(N-méthoxyacétyl-amino)-2,4,6-tribromo-N-bis (β-acétoxyéthyl)-N'-(2-méthyl-1,3-dihydroxy-2- propyl) isophtalamide

6,1 g (0,003 mole) du composé obtenu en (a) sont mis en solution dans 30 ml de DMAC en présence de 2 ml (0,015 mole) de triéthylamine. La solution est chauffée à 50°C. On ajoute goutte à goutte 1,9 g (0,018 mole) de 2-amino-2-méthyl-propane-1,3-diol en solution dans 15 ml de DMAC.

Après agitation 48 heures à 50°C la solution est évaporée à sec. L'huile brute obtenue est directement utilisée pour la saponification finale.

CCM : Toluène/MEC/acide formique (60/25/25)

Rf : 0,25.

c) Préparation du 5-(N-méthoxyacétyl-amino)-2,4,6-tribromo-N-bis-(β-hydroxyéthyl)-N'-(2-méthyl-1,3-dihydroxy-2-propyl) isophtalamide

Le composé obtenu en (b) est saponifié dans la soude aqueuse 2N (1,3 équivalent). Après agitation 24 heures à température ambiante, les produits réactionnels sont passés successivement sur résine cationique, puis anionique.

CCM : Toluène/MEC/acide formique (60/25/25) Rf : 0,1.

EXEMPLE 9

Préparation du L-5-(N-2-hydroxypropionyl-amino)-2,4,6-tribromo-N-bis (β-hydroxyéthyl)-N'-(2-méthyl-1,3-dihydroxy--2- propyl) isophtalamide

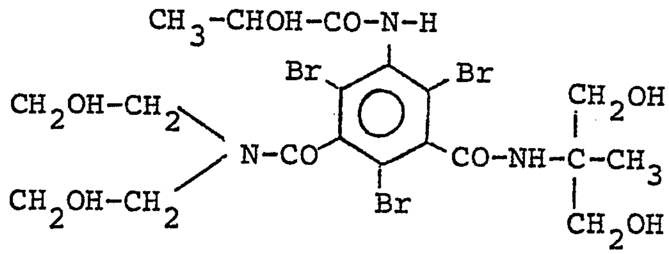

a) Préparation du chlorure d'acide 3-N-bis (acétoxyéthyl) carbamoyl-5-(L-2-acétoxypropionyl-amino)-2,4,6-tribromo isophtalique

Il est préparé comme décrit à l'exemple 8(a)

Rendement : 85 %.

CCM : Toluène/MEC/HCOOH (60/25/25) Rf: 0,6

Structure confirmée en IR et RMN.

b) Préparation du L-5-(N-2-acétoxypropionyl-amino)-2,4,6-tribromo-N-bis (β-acétoxyéthyl)-N'-(2-méthyl-1,3-dihydroxy-2-propyl) isophtalamide

On obtient ce composé à partir du composé obtenu en (a), comme décrit à l'exemple 8(b). Rendement : 60 %.

CCM : Butanol/eau/$CH_3COOH$ (50/25/11) Rf : 0,86.

c) Préparation du L-5-(N-2-hydroxypropionyl-amino)-2,4,6-tribromo-N-bis (β-hydroxyéthyl)-N'-(2-méthyl-1,3-dihydroxy-2- propyl) isophtalamide

On saponifie le composé obtenu en (b) comme décrit à l'exemple 8(c).

CCM : Butanol/eau/$CH_3COOH$ (50/25/11) Rf : 0,41.

EXEMPLE 10

Préparation du L-5-(N-2-hydroxy propionyl-amino)-2,4,6-tribromo-N,N'-bis (1,3-dihydroxy-2-propyl) isophtalamide

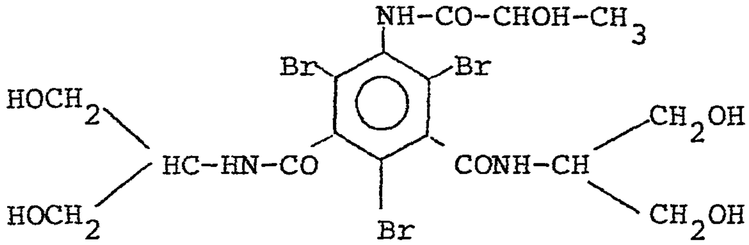

a) Préparation du L-5-(N-2-acétoxy propionyl amino)-2,4,6-tribromo-N,N'-bis (1,3 dihydroxy-2-propyl) isophtalamide

On opère comme dans l'exemple 4b en utilisant à la place du 2-amino-2-méthyl propane-1,3-diol le sérinol (2-amino-propane-1,3-diol)

On obtient un produit solide blanc.

Rendement : 90 %

CCM: Butanol/ $H_2O$ / acide acétique (50/25/11)

Rf = 0,70

b) Préparation du L-5-(N-2-hydroxy propionyl-amino)-2,4,6-tribromo-N,N'-bis (1,3-dihydroxy-2-propyl) isophtalamide

On traite le composé obtenu en 10(a) comme décrit dans l'exemple 4(c)

On obtient un produit solide blanc.

Rendement: 80 %

CCM: Butanol/$H_2O$/acide acétique (50/25/11)

Rf = 0,42

EXEMPLE 11

Préparation du 5-(N-hydroxyacétyl-amino)-2,4,6-tribromo-N-(β-hydroxyéthyl)-N'-(2-méthyl-1,3-dihydroxy-2-propyl) isophtalamide

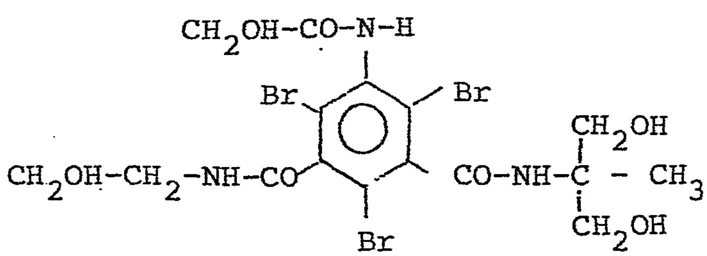

a) Préparation du chlorure d'acide 3-N-acétoxyéthyl carbamoyl-5-N-(acétoxyacétyl-amino)-2,4,6-tribromo isophtalique

Il est préparé comme décrit à l'exemple 8(a), en utilisant le chlorure d'acide acétoxyacétique. Rdt: 60 %.
CCM : Toluène/MEC/HCOOH (60/25/25) Rf : 0,72.

Structure confirmée en IR et RMN.

b) Préparation du 5-N(acétoxyacétyl-amino)-2,4,6-tribromo-N-(acétoxyéthyl)-N'-(2-méthyl-1,3-dihydroxy-2 propyl) isophtalamide

Il est préparé à partir du composé obtenu en (a) comme décrit à l'exemple 8(b). Rendement : 48,5 %.
CCM : Toluène/MEC/HCOOH (60/25/25) Rf : 0,34.

c) Préparation du 5-(N-hydroxyacétyl-amino)-2,4,6-tribromo-N-(β-hydroxyéthyl)-N'-(2-méthyl-1,3-dihydroxy-2-propyl) isophtalamide

Il est obtenu par saponification du composé obtenu en (b), comme décrit à l'exemple 8(c).
CCM : Butanol/eau/$CH_3COOH$ (50/25/11) Rf : 0,58.

EXEMPLE 12

Préparation du 5-(N-2-hydroxypropionyl-amino)-2,4,6-tribromo-N-(β-hydroxyéthyl)-N'-(2-méthyl-1,3-dihydroxy-2-propyl) isophtalamide

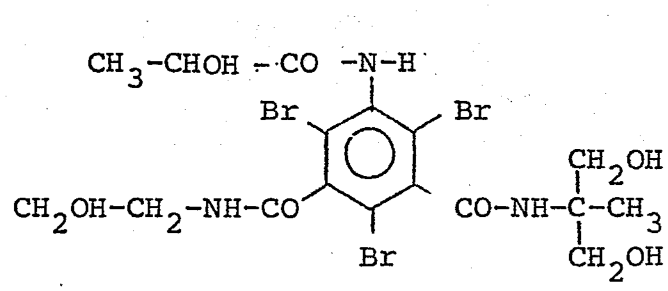

a) Préparation du chlorure d'acide 3-N-acétoxyéthyl-carbamoyl-5-N-(2-acétoxypropionyl-amino)-2,4,6-tribromo-isophtalique

Il est préparé comme décrit à l'exemple 8(a), en utilisant le chlorure d'acide 2-acétoxypropionique.
CCM : Toluène/MEC/HCOOH (60/25/25) Rf : 0,62.

Structure confirmée en RMN.

b) Préparation du 5-N-(2-acétoxypropionyl-amino)-2,4,6-tribromo-N-(acétoxyéthyl)-N'-(2-méthyl-1,3-dihydroxy-2-propyl) isophtalamide

Il est préparé à partir du composé obtenu en (a), comme décrit à l'exemple 8(b). Rendement : 73 %.

CCM : Toluène/MEC/HCOOH (60/25/25) Rf : 0,25.

Structure confirmée en RMN.

c) Préparation du 5-(N-2-hydroxypropionyl-amino)-2,4,6-tribromo-N-(β-hydroxyéthyl)-N'-(2-méthyl-1,3-dihydroxy-2-propyl) isophtalamide

Il est obtenu par saponification du composé obtenu en (b), comme décrit à l'exemple 8(c). Rendement: 82 %.

CCM : Toluène/MEC/HCOOH (60/25/25) Rf : 0,1

Butanol/eau/$CH_3COOH$ (50/25/11) Rf : 0,55

EXEMPLE 13

Préparation du L-5-(N-α-hydroxypropionyl-amino-acétyl-amino)-2,4,6-tribromo-N,N'-bis-/bis (β-hydroxyéthyl)7 isophtalamide

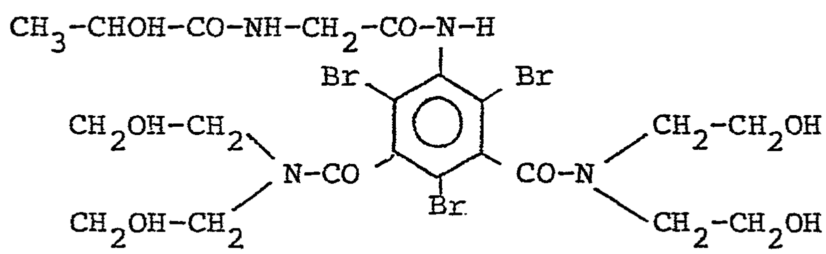

a) Préparation du L-5-(N-α-acétoxypropionyl-amino-acétyl-amino)-2,4,6-tribromo-N,N'-bis /bis (β-hydroxyéthyl)7 isophtalamide

Il est préparé comme décrit à l'exemple 6(b) par réaction du chlorure de l'acide L-α-acétoxy propionique sur le 5-aminoacétylamino-2,4,6-tribromo-N,N'-bis /bis (β-hydroxyéthyl)7 isophtalamide.

CCM: Isobutanol/isopropanol/ammoniac (35/35/40) Rf: 0,75.

b) Préparation du L-5-(N-α-hydroxypropionyl-amino-acétyl-amino)-2,4,6-tribromo-N,N'-bis-/bis (β-hydroxyéthyl)7 isophtalamide

Il est obtenu par saponification du composé obtenu en (a), comme décrit à l'exemple 6(c).

CCM : Chloroforme/acétone/acide acétique (40/60/20)

Rf = 0,1

EXEMPLE 14

Préparation du 5-acétamido-2,4,6-tribromo-N,N'-bis (N-méthyl-N-2,3,4,5,6-penta hydroxy hexyl) isophtalamide

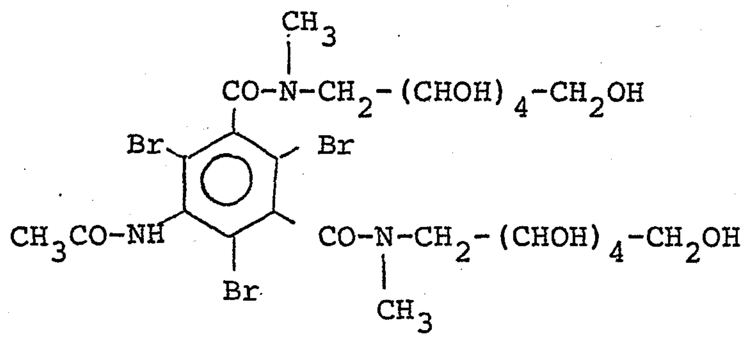

a) Préparation du 5-amino-2,4,6-tribromo-N,N'-bis (N-méthyl-N-2,3,4,5,6-penta hydroxy hexyl) isophtalamide

86 g de N-méthyl glucamine en solution aqueuse (100ml) sont ajoutés goutte à goutte dans une solution de 100g de chlorure d'acide 2,4,6 tribromo amino isophtalique dans 500 ml de dioxanne, en présence de 44 g de $NHCO_3$.

La réaction est terminée après 16 h d'agitation à température ambiante.

Le produit brut est obtenu par évaporation à sec et lavage à l'éthanol.

Rendement : 80 %

CCM : Toluène/MEC/HCOOH (60/25/25) : Rf = O

Isopropanol/Isobutanol/ammoniaque (35/35/40) Rf= 0,5

b) Préparation du 5-acétamido-2,4,6-tribromo-N,N'-bis (N-méthyl-N-2,3,4,5,6 - penta acétoxy hexyl) isophtalamide.

A 0,176 mole du produit précédent dissous dans 500 ml de dioxanne anhydre, on ajoute goutte à goutte 3 moles de chlorure d'acétyle et on chauffe 16 h à 70°C.

Après élimination des sels minéraux par filtration, la solution est versée dans 2 l d'eau. Le précipité obtenu est filtré, lavé à l'eau et séché.

Rendement : 42 %

Pureté : 101 % en brome

CCM : Isobutanol/Isopropanol/ammoniaque (35/35/40) Rf= 0,65

c) Préparation du 5-acétamido-2,4,6-tribromo-N,N'-bis-(N-méthyl-N-2,3,4,5,6-penta hydroxy hexyl) isophtalamide

10g du produit acétylé précédent (0,08 mole) sont dissous dans 60 ml de solution de soude 2N et agités 4 h à température ambiante.

Le liquide réactionnel est dilué à l'eau et déminéralisé sur résines échangeuses d'ions.

On obtient le produit final par évaporation à sec et lavage à l'éthanol.

Rendement : 80 %

Pureté : 98,2 % d'après le dosage de brome

CCM : Isobutanol/Isopropanol/$NH_4OH$ (35/35/40) Rf = 0,65.

EXEMPLE 15

Préparation du 5,5'-/N,N'-(hydroxy-2-éthyl) malonyldiimino/ bis /2,4,6-tribromo-N,N'-bis-(2-méthyl-1,3-dihydroxy-2-propyl)/ isophtalamide

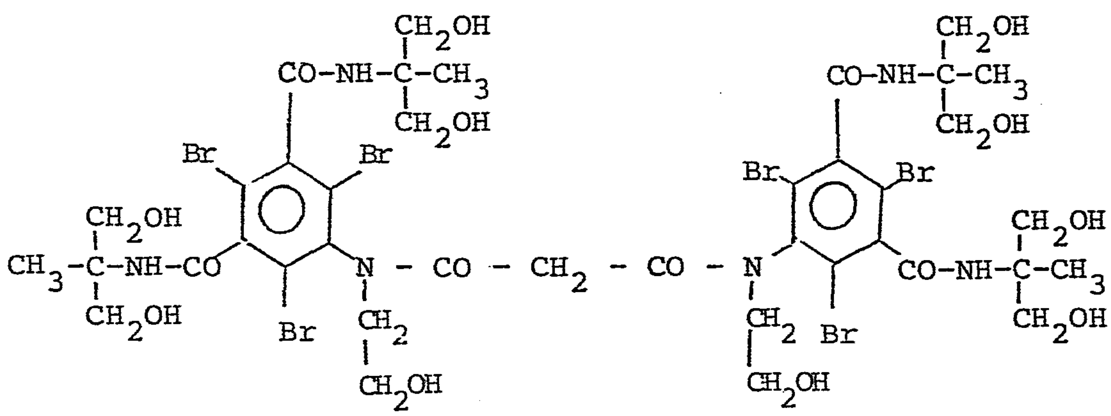

a) Préparation du chlorure de l'acide 5,5'-malonyldiimino-bis (2,4,6-tribromo isophtalique)

A 90 g (0,2 mole) de chlorure d'acide 5-amino-2,4,6-tribromo isophtalique dissous dans 500 ml de toluène, on ajoute goutte à goutte, à 80°C, une solution de 0,12 mole de chlorure d'acide malonique dans 200 ml de toluène. Le mélange réactionnel est chauffé 1 heure à 80°C, puis agité 16 heures à température ambiante. Le produit qui précipite peu à peu est séparé par essorage et lavé au cyclohexane. Un deuxième jet est obtenu par addition de 200 ml de cyclohexane aux eaux mères. On obtient 68,5 g de chlorure d'acide. Rendement 70 %.

CCM : après condensation avec la propylamine

| | | |
|---|---|---|
| Butanol/eau/$CH_3COOH$ | (50/25/11) | Rf : 0,68 |
| Toluène/méthyléthylcétone/HCOOH | (60/25/25) | Rf : 0,57 |
| Isobutanol/isopropanol/$NH_4OH$ | (35/35/40) | Rf : 0,75 |

b) <u>Préparation du 5,5'-malonyldiimino-bis /2,4,6-tribromo-N,N'-bis-(2-méthyl-1,3-dihydroxy-2-propyl) isophtalamide/</u>

On ajoute peu à peu 9 g (0,1 mole) de 2-amino 2-méthylpropane-1,3-diol à une solution de 19,6 g (0,02 mole) du chlorure d'acide obtenu en (a) dans 40 ml de diméthylacétamide en présence de 0,1 mole de triéthylamine. Après 16 heures d'agitation à température ambiante, on élimine le chlorhydrate de triéthylamine par filtration et la solution obtenue est ajoutée goutte à goutte à 200 ml d'acétone. Après essorage, lavage à l'acétone, le produit est séché à l'étuve à 70°C. On obtient 15 g de solide blanc. Rendement: 60 %.

| | | | |
|---|---|---|---|
| CCM : | Butanol/eau/$CH_3COOH$ | (50/25/11) | Rf : 0,55 |
| | Isobutanol/isopropanol/$NH_4OH$ | (35/35/40) | Rf : 0,45 |
| | Chloroforme/méthanol | (60/40) | Rf : 0,75 |

c) <u>Préparation du 5,5'-/N,N'-(hydroxy-2-éthyl) malonyl-diimino/bis /2,4,6-tribromo-N,N'-bis-(2-méthyl-1,3-dihydroxy-2-propyl) isophtalamide/</u>

On prépare 0,144 mole de méthylate de sodium à partir de 3,3 g (0,144 mole) de sodium dans 60 ml de méthanol, puis on ajoute 130 ml de propylèneglycol-1,2.

On verse dans cette solution 56,3 g (0,045 mole) du produit obtenu en (b), puis 300 ml de méthanol. On chauffe à 50°C. Après 30 mn, la solution est limpide et on évapore sous vide le méthanol en excès.

On ajoute à la solution obtenue, à 50°C et sous vive agitation, 9 ml (0,135 mole) de 2-chloroéthanol et on agite 4 heures à 50°C. La solution refroidie à température ambiante est versée goutte à goutte dans 2000 ml d'acétone. Le précipité est filtré, lavé à l'acétone, séché sous vide. Le produit obtenu est dissous dans 500 ml d'eau. La solution est déminéralisée par élution sur résine cationique, puis résine anionique. Après décoloration sur charbon actif une heure à température ambiante, la solution est évaporée sous vide. On obtient 51 g de produit. Rendement : 85 %.

CCM : Butanol/eau/acide acétique (50/25/11) Rf : 0,65
Isopropanol/isobutanol/ammoniaque (35/35/40) Rf: 0,55.

EXEMPLE 16

Préparation du 5-méthoxyacétyl-amino-2,4,6-tribromo-N-2,3-dihydroxypropyl-N'-méthyl-N'-2,3-dihydroxypropyl isophtalamide.

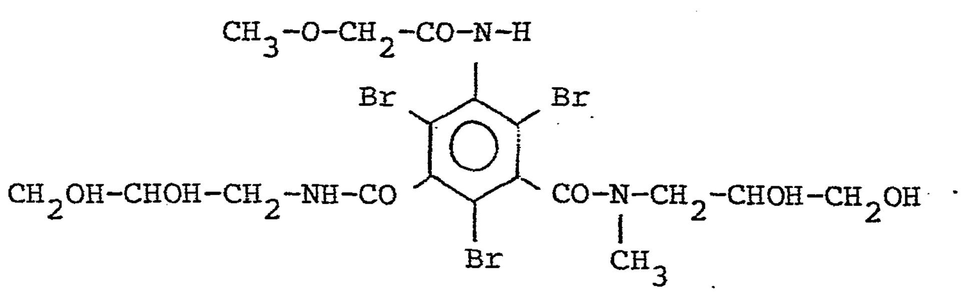

a) Préparation du chlorure de l'acide 5-méthoxyacétyl-amino-2,4,6-tribromo-N-2,3-dihydroxypropyl-carbamoyl benzoïque

Un mélange contenant 3 ml ( 0,022 mole) de triéthylamine et 1,8 g ( 0,02 mole) de 2,3-dihydroxypropylamine et dissous dans 30 ml de D M A C est additionné goutte à goutte à température ambiante sur une

solution de 10,5 g (0,02 mole) du dichlorure d'acide 5-méthoxyacétyl-amino-2,4,6-tribromo isophtalique dans 30 ml de DMAC.

L'agitation est maintenue une nuit à 40°C. Le chlorhydrate de triéthylamine est filtré et le solvant évaporé sous vide. Le produit est cristallisé dans l'éther.
Rendement : 75 %.
CCM : Toluène/MEC/HCOOH (60/25/25) Rf : 0,30.

b) <u>Préparation du 5-méthoxyacétyl-amino-2,4,6-tribromo-N-2,3-dihydroxypropyl-N'-méthyl-N'-2,3-dihydroxypropyl isophtalamide</u>

7,5 g (0,013 mole) du composé obtenu en (a) sont dissous dans 20 ml de DMAC. On ajoute à température ambiante une solution de 1,35 g (0,013 mole) de N-méthyl-2,3-dihydroxypropylamine et de 1,8 g (0,018 mole) de triéthylamine.

CCM : Butanol/eau/acide acétique (50/25/11) Rf : 0,40.
$CH_2Cl_2$/méthanol (80/25) Rf : 0,37

## REVENDICATIONS

1. Composés choisis parmi les composés de formule

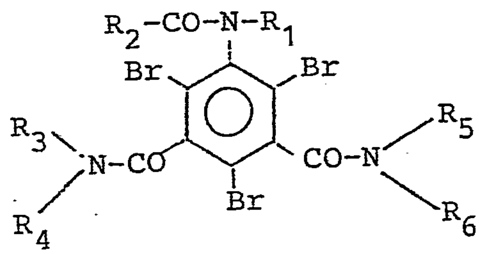

(Ia)

et les composés de formule

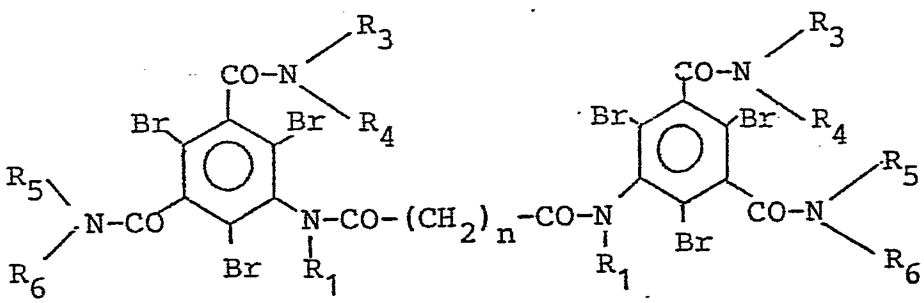

(Ib)

dans lesquelles

$R_1$ représente un atome d'hydrogène ou un groupe alcoyle en $C_1$ à $C_6$ mono- ou polyhydroxylé,

$R_2$ représente un groupe méthyle, un groupe méthoxy méthyle, un groupe alcoyle en $C_1$ à $C_6$ mono- ou polyhydroxylé ou un groupe (alcoyl en $C_{1-6}$ mono- ou polyhydroxylé) carbonylamino méthyle,

$R_3$ et $R_5$ représentent indépendamment l'un de l'autre un groupe alcoyle en $C_1$ à $C_6$ mono- ou polyhydroxylé, et

$R_4$ et $R_6$ représentent indépendamment l'un de l'autre un atome d'hydrogène, un groupe alcoyle en $C_1$ à $C_6$ ou un groupe alcoyle en $C_1$ à $C_6$ mono- ou polyhydroxylé, et

$\underline{n}$ est un nombre entier de 1 à 6.

2. Le 5-(N-2,3-dihydroxypropyl N-acétylamino)-2,4,6-tribromo-N,N'-bis (2,3-dihydroxypropyl) isophtalamide.

3. Le 5-(N-2,3-dihydroxy propyl-N-méthoxyacétylamino)-2,4,6-tribromo-N,N'-bis (2,3-dihydroxypropyl) isophtalamide.

4. Produit opacifiant comprenant un composé selon l'une quelconque des revendications 1 à 3.

5. Produit opacifiant selon la revendication 4, caractérisé en ce qu'il comprend en outre un composé iodobenzénique.

6. Produit opacifiant selon la revendication 5, caractérisé en ce que les composés bromobenzéniques et iodobenzéniques sont présents dans un rapport molaire de 1/2 à 2/1.

7. Produit opacifiant selon l'une quelconque des revendications 4 à 6 sous forme de solution aqueuse.

REVENDICATIONS

1. Procédé de préparation de composés choisis parmi les composés de formule

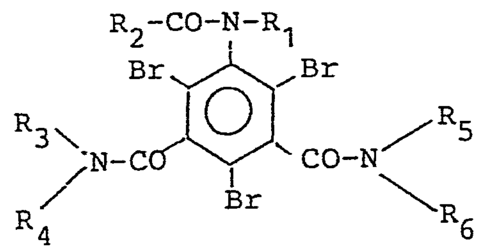

(Ia)

et les composés de formule

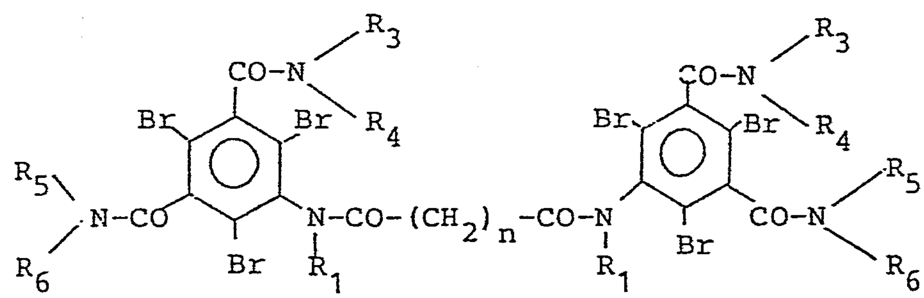

(Ib)

dans lesquelles

$R_1$ représente un atome d'hydrogène ou un groupe alcoyle en $C_1$ à $C_6$ mono- ou polyhydroxylé,

$R_2$ représente un groupe méthyle, un groupe méthoxy méthyle, un groupe alcoyle en $C_1$ à $C_6$ mono- ou polyhydroxylé ou un groupe (alcoyl en $C_{1-6}$ mono- ou polyhydroxylé) carbonylamino méthyle,

$R_3$ et $R_5$ représentent indépendamment l'un de l'autre un groupe alcoyle en $C_1$ à $C_6$ mono- ou polyhydroxylé, et

$R_4$ et $R_6$ représentent indépendamment l'un de l'autre un atome d'hydrogène, un groupe alcoyle en $C_1$ à $C_6$ ou un groupe alcoyle en $C_1$ à $C_6$ mono- ou polyhydroxylé, et

n est un nombre entier de 1 à 6,

caractérisé en ce que l'on prépare les composés de formules Ia et Ib par des réactions d'acylation et/ou d'alcoylation de composés à groupe(s) amino.

2. Procédé selon la revendication 1, caractérisé en ce que pour préparer des composés de formule Ia dans lesquels les groupes $-NR_3R_4$ et $-NR_5R_6$ sont identiques,

1 - on effectue une acylation du composé de formule

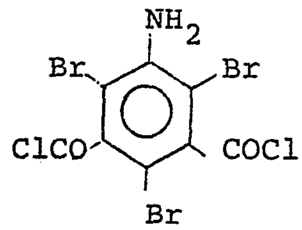

(II)

avec un chlorure d'acide de formule

$$R'_2 - COCl \quad (III)$$

$R'_2$ étant un groupe $R_2$ dont les groupes hydroxy éventuels sont protégés, conduisant à un dichlorure d'acide de formule

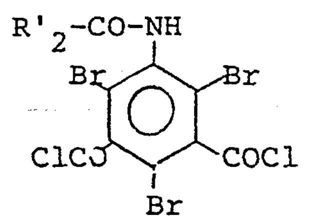

(IVa)

puis l'on effectue une condensation du dichlorure d'acide ainsi obtenu avec une amine de formule

$$R_3R_4NH \quad (V)$$

$R_3$ et $R_4$ ayant la définition donnée ci-dessus , conduisant à un composé de formule

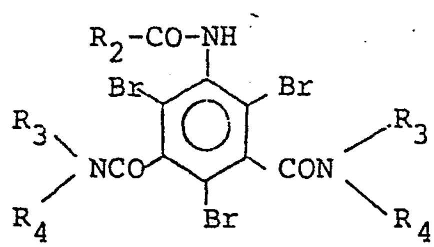

(VIa)

ou bien

2 - on effectue une condensation du chlorure d'acide (II) avec une amine de formule (V) conduisant à une amine de formule

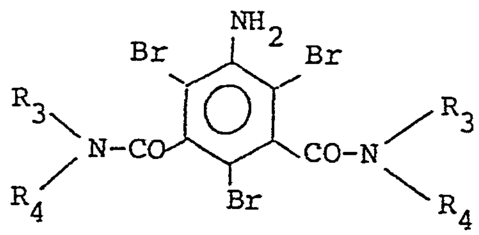

(VIII a)

puis une acylation de l'amine ainsi obtenue avec un chlorure d'acide de formule (III) conduisant à un composé de formule (VIa),

3 - on effectue éventuellement une alcoylation du composé de formule (VIa) avec un dérivé chloré de formule

$$Cl - R_1 \qquad (VII)$$

$R_1$ ayant la signification donnée ci-dessus, et/ou une réaction de déprotection.

3. Procédé selon la revendication 1, caractérisé en ce que pour préparer les composés de formule (Ia) dans lesquels les groupes $-NR_3R_4$ et $-NR_5R_6$ sont différents

1 - on effectue une acylation d'un composé de formule

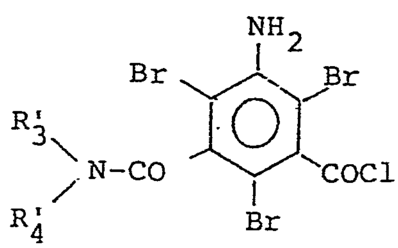

(IX)

$R'_3$ et $R'_4$ étant des groupes $R_3$ et $R_4$ dont les groupes hydroxy sont protégés, avec un chlorure d'acide de formule (III)

$$R'_2 - COCl \qquad (III)$$

conduisant à un chlorure d'acide de formule

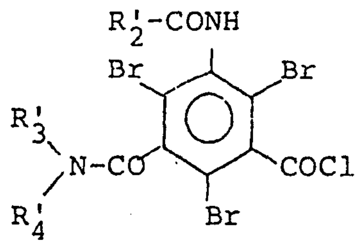

(Xa)

puis une condensation de ce chlorure d'acide (Xa) avec une amine de formule $R_5R_6NH$ (XI), conduisant à un composé de formule

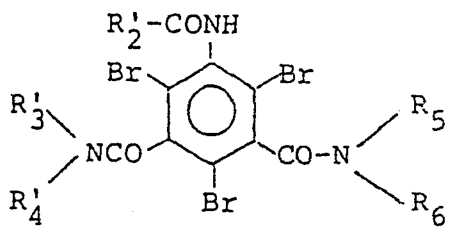

(XIIa)

ou bien

2 - on effectue une condensation d'un composé de formule (IX) avec une amine de formule (XI) conduisant à une amine de formule

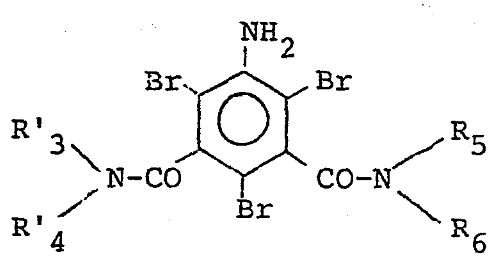

(XIII)

puis une acylation de l'amine de formule (XIII) avec un chlorure d'acide de formule (III) conduisant à un composé de formule (XIIa), ou bien

3 - on effectue une acylation d'un composé de formule (II)

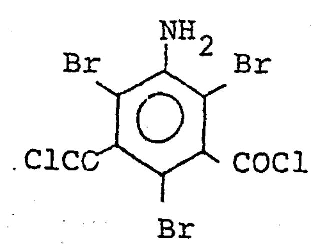

(II)

avec un chlorure d'acide de formule (III), conduisant à un chlorure d'acide de formule

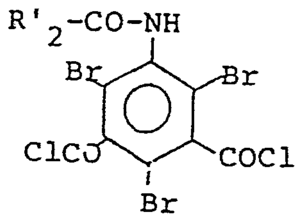

(IVa)

puis successivement une condensation avec une amine de formule (V)

$R_3R_4NH$ (V)

et une condensation avec une amine de formule (XI),

4 - on effectue éventuellement une alcoylation du composé de formule (XIIa) avec un dérivé chloré de formule (VII) et/ou une réaction de déprotection.

4. Procédé selon la revendication 1, caractérisé en ce que pour préparer les composés de formule (Ib) dans laquelle les groupes $-NR_3R_4$ et $-NR_5R_6$ sont identiques on effectue une acylation du composé de formule (II)

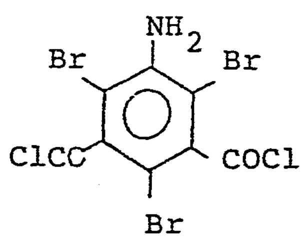

(II)

avec un chlorure d'acide de formule

$Cl-CO-(CH_2)_n-CO-Cl$ (IIIb)

conduisant à un chlorure d'acide de formule

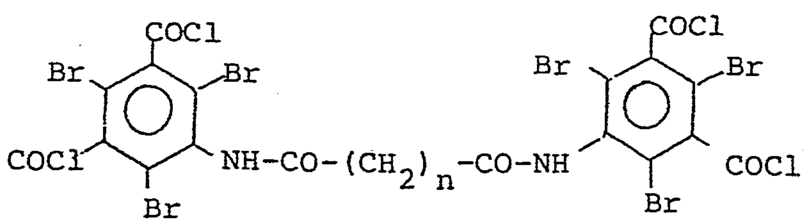

(IVb)

puis une condensation de ce chlorure d'acide (IVb) avec une amine de formule (V)

$R_3R_4NH$ (V)

conduisant à un composé de formule

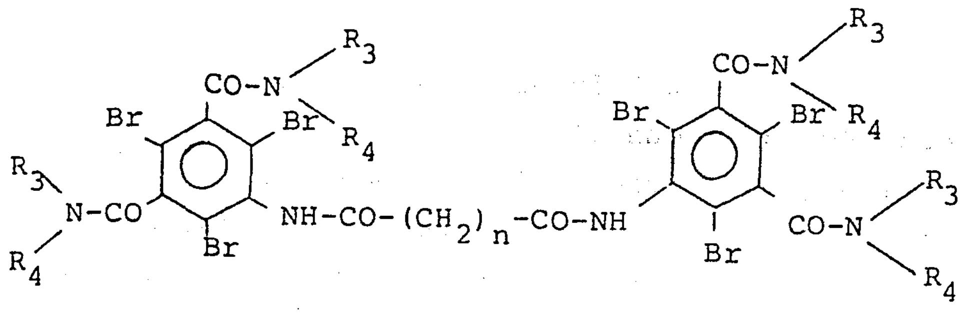

(VIb)

cette réaction étant suivie éventuellement d'une alcoylation avec un dérivé chloré de formule (VII)

$$Cl - R_1 \quad (VII)$$

5. Procédé selon la revendication 1, caractérisé en ce que pour préparer les composés de formule (Ib) dans lesquels les groupes $-NR_3R_4$ et $-NR_5R_6$ sont différents on effectue une condensation d'un composé de formule (IX)

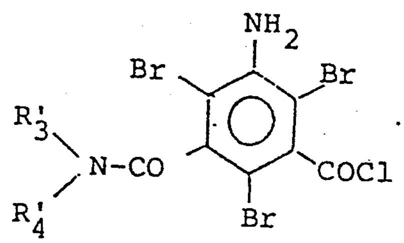

(IX)

avec un chlorure d'acide de formule (IIIb)

$$Cl - CO - (CH_2)_n - CO - Cl \quad (IIIb)$$

puis une condensation du chlorure d'acide obtenu avec une amine de formule (XI)

$$R_5R_6NH \quad (XI)$$

cette réaction étant suivie éventuellement d'une alcoylation avec un dérivé chloré de formule (VII)

$$Cl - R_1 \quad (VII)$$

et/ou d'une réaction de déprotection.

6. Procédé de préparation de produits opacifiants, caractérisé en ce que l'on met un composé de formules Ia ou Ib, telles que définies à la revendication 1, sous une forme convenant pour une administration chez l'homme.

7. Procédé de préparation de produits opacifiants selon la revendication 6, caractérisé en ce que l'on met un composé bromobenzénique de formule Ia ou Ib, telles que définies à la revendication 1, en mélange avec un composé iodobenzénique sous une forme convenant pour une administration chez l'homme.

Office européen des brevets

# RAPPORT DE RECHERCHE EUROPEENNE

0118348

Numéro de la demande

EP 84 40 0314

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | US-A-3 655 752 (J.H. ACKERMANN et al.)<br>* Revendications * | 1,4-7 | C 07 C 103/76<br>C 07 C 103/78<br>A 61 K 49/04 |
| Y | FR-A-2 355 808 (SCHERING AG)<br>* Revendications * | 1,4-7 | |
| Y | EP-A-0 015 867 (SCHERING AG)<br>* Revendications * | 1,4-7 | |
| Y | EP-A-0 049 745 (SCHERING AG)<br>* Revendications * | 1,4-7 | |
| P,X | EP-A-0 073 715 (GUERBET)<br>* Revendications * | 1-7 | |

DOMAINES TECHNIQUES RECHERCHES (Int. Cl. 3)

C 07 C 103/00
A 61 K 49/00

Le présent rapport de recherche a été établi pour toutes les revendications

| Lieu de la recherche | Date d'achèvement de la recherche | Examinateur |
|---|---|---|
| LA HAYE | 22-05-1984 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82